# EUROPÄISCHE PATENTSCHRIFT

(11) **EP 3 646 029 B1**
(45) Veröffentlichungstag und Bekanntmachung des Hinweises auf die Patenterteilung: **25.05.2022**
(21) Anmeldenummer: 18731411.7
(22) Anmeldetag: 12.06.2018
(51) Int. Cl.: G01N 33/543, G01N 33/574, B01L 3/00, G01N 33/487

(54) **DETEKTIONSSYSTEM UND VERFAHREN ZU DESSEN HERSTELLUNG**
DETECTION SYSTEM AND PROCESS FOR ITS PRODUCTION
SYSTÈME DE DÉTECTION ET PROCÉDÉ POUR SA FABRICATION

(30) Priorität: 28.06.2017 DE 102017114349
(43) Veröffentlichungstag der Anmeldung: 06.05.2020
(73) Patentinhaber: Technische Universität Darmstadt, 64289 Darmstadt (DE)
(72) Erfinder: EL KHOURY, Mario, 64285 Darmstadt (DE); ENSINGER, Wolfgang, 64839 Münster-Altheim (DE); GÖRINGER, H. Ulrich, 64380 Roßdorf (DE); QUEDNAU, Sebastian, 64665 Alsbach-Hähnlein (DE); DUZNOVIC, Ivana, 60594 Frankfurt (DE); SCHLAAK, Helmut F., 64372 Ober-Ramstadt (DE)
(74) Vertreter: LifeTech IP Spies & Behrndt Patentanwälte PartG mbB
(86) Internationale Anmeldenummer: PCT/EP2018/065542
(87) Internationale Veröffentlichungsnummer: WO 2019/001952

(56) Entgegenhaltungen:
- WO-A1-2015/172992
- US-A1- 2010 243 449
- US-A1- 2012 142 016
- WOLFGANG ENSINGER ET AL: "The iNAPO Project: Biomimetic Nanopores for a New Generation of Lab-on-Chip Micro Sensors", PROCEEDINGS OF THE 2ND WORLD CONGRESS ON RECENT ADVANCES IN NANOTECHNOLOGY, 1. April 2017 (2017-04-01), XP055500092, ISSN: 2371-5308, DOI: 10.11159/icnnfc17.141 ISBN: 978-1-927877-28-9
- Mario El Khoury ET AL: "Integration of Nanochannels for Lab-on-Chip-Systems", Micro-Nano-Integration; 6. GMM Workshop Duisburg 5-6 October 2016, 8. Dezember 2016 (2016-12-08), XP055500095, ISBN: 978-3-8007-4278-3 Gefunden im Internet: URL:https://ieeexplore.ieee.org/iel7/77760 50/7776051/07776085.pdf [gefunden am 2018-08-15]
- YOHAI MANDABI ET AL: "Label-free DNA detection using the narrow side of funnel-type etchednanopores", BIOSENSORS AND BIOELECTRONICS, Bd. 42, 1. April 2013 (2013-04-01), Seiten 362-366, XP055500097, AMSTERDAM, NL ISSN: 0956-5663, DOI: 10.1016/j.bios.2012.10.036
- ALZGHOUL SALAH ET AL: "Measurement of serum prostate cancer markers using a nanopore thin film based optofluidic chip", BIOSENSORS AND BIOELECTRONICS, ELSEVIER SCIENCE LTD. UK, AMSTERDAM, NL, Bd. 77, 9. Oktober 2015 (2015-10-09), Seiten 491-498, XP029311827, ISSN: 0956-5663, DOI: 10.1016/J.BIOS.2015.10.006
- BEI LIU ET AL: "Detection of the human prostate-specific antigen using an aptasensor with gold nanoparticles encapsulated by graphitized mesoporous carbon", MICROCHIMICA ACTA ; AN INTERNATIONAL JOURNAL ON MICRO AND TRACEANALYSIS, SPRINGER-VERLAG, VI, Bd. 178, Nr. 1 - 2, 8. Mai 2012 (2012-05-08), Seiten 163-170, XP035075079, ISSN: 1436-5073, DOI: 10.1007/S00604-012-0822-5
- ARMAGAN KOCER ET AL: "Nanopore sensors: From hybrid to abiotic systems", BIOSENSORS AND BIOELECTRONICS, ELSEVIER SCIENCE LTD. UK, AMSTERDAM, NL, Bd. 38, Nr. 1, 12. Mai 2012 (2012-05-12), Seiten 1-10, XP028398315, ISSN: 0956-5663, DOI: 10.1016/J.BIOS.2012.05.013 [gefunden am 2012-06-19]

## Beschreibung

Die vorliegende Erfindung bezieht sich auf ein Detektionssystem und ein Verfahren zu dessen Herstellung und insbesondere auf die Detektion von (Bio-) Molekülen (Analyte, Liganden, etc.) in biotischen und abiotischen Systemen.

### Hintergrund

Trotz enormer biomedizinischer Forschungsanstrengungen sind Krebserkrankungen nach wie vor mit hohen Sterblichkeitsraten behaftet. Zudem stellt jede Krebsart eine therapeutische und diagnostische Herausforderung an die behandelnden Ärzte dar. Verlässliche Voraussagen, inwieweit sich der Verlauf einer Krankheit im Kontext der angewendeten Therapiemaßnahmen entwickeln wird, sind leider immer noch mit hohen Fehlerquoten behaftet. In diesem Kontext kommt frühdiagnostischen Messmethoden eine große Bedeutung zu.

Prostatakrebs (PCa) ist beispielsweise die in der westlichen Welt bei Männern am häufigsten vorkommende Krebserkrankung. Allein in Deutschland werden pro Jahr etwa 63.000 Neuerkrankungen diagnostiziert. Eine schnelle Vor-Ort-Diagnostik, die präzise zu einem aussagekräftigen Ergebnis kommt, sollte die Heilungschancen für PCa-Patienten deutlich erhöhen und dadurch in vielen Fällen lebensrettend sein. Zur frühzeitigen Detektion von PCa kann die Konzentration des Prostata-spezifischen Antigens (PSA) gemessen werden. Hierbei handelt es sich um ein glykosyliertes Eiweiß, dass im Blutserum detektiert werden kann. Der Nachweis von PSA greift im Routinebetrieb vornehmlich auf Antikörper-basierte Detektionsverfahren zurück, die neben hohen Kosten häufig auch falsch-positive Resultate produzieren. Dieser Umstand ist Ausgangspunkt für die Suche nach alternativen Messverfahren zur Detektion von PSA.

Ein Ansatz basiert auf Nukleinsäure-Biopolymere wie Aptamere, die in der Lage sind, PSA spezifisch zu erkennen und hochaffin zu binden. Sie können in einem handlichen Sensorsystem eingesetzt werden, um die PSA-Konzentration im Blut zu messen und somit ein mögliches Prostatakarzinom zu detektieren. Werden solche Aptamere an die Innenwand nanoskaliger Poren/Kanäle einer Filterfolie fixiert, kann ein einfacher Sensor zur PSA-Detektion hergestellt werden.

Ein Beispiel für diese Art von Detektion findet sich in der Veröffentlichung: Ali M, Nasir S, Ensinger W.: "Bioconjugation-induced ionic current rectification in aptamer-modified single cylindrical Pores"; Chem Commun 2015, 51: 3454-3459. Hierbei wird eine Potentialdifferenz zwischen beiden Seiten einer Kunststofffolie angelegt, um so einen messbaren Ionenstrom zu erzeugen. Beinhaltet das Blutserum die zu detektierende Biomoleküle, so werden diese Moleküle an den Aptameren in den Poren gebunden, was zu einer Verengung der Querschnittsfläche der Poren führt. Dadurch erhöht sich der elektrische Widerstand der einzelnen Poren in Abhängigkeit von einer Konzentration der Aptamer-Komplexe. Folglich lässt sich über die Abnahme des gemessenen Ionenstroms direkt auf die Konzentration des Biomoleküls schließen. Außerdem offenbart M. El Khoury et al.: "Integration of Nanochannels on Lab-on-Chip-Systems" eine bekannte Integration von Nanokanälen in einem sogenannten Lab-on-Chip-System und ein entsprechendes Herstellungsverfahren und W. Ensinger et al "The iNAPO Project: Biomimetic Nonopores for a New Generation of Lab-on-Chip Micro Sensors" offenbart ein weiteres konventionelles Detektionsystem basierend auf Nonoporen.

Bei der Herstellung dieser Sensoren werden an einer Multiporen-Folie - insbesondere im Bereich der Poren - die genutzten Aptameren aufgebracht. Dieser Schritt wird als Funktionalisieren bezeichnet, da die resultierende Folie dadurch für eine bestimmte Funktion (Detektion eines Biomoleküls) vorbestimmt ist. Bei dem bisher genutzten Herstellungsverfahren wurde die Folie zunächst funktionalisiert, dann zurückgeschnitten und schließlich in einem gewünschten Detektionsbereich angeordnet. Dieses sogenannte "pick-and-place"-Verfahren ist aufwendig und ist nur bedingt automatisierbar. Funktionalisierte Membranen können während des Integrationsprozesses (Einbaus) beschädigt werden und dadurch ihre Funktionalität wieder verlieren. Daher bietet die Funktionalisierung der geätzten Membran nach deren Integration Vorteile.

Daher besteht ein Bedarf nach einem verbesserten Herstellungsverfahren für diese Sensoren. Außerdem besteht ein Bedarf an verbesserten Aptameren, die hoch-sensitiv auf das PSA sind und somit die Resultate deutlich verbessern.

### Zusammenfassung

Zumindest ein Teil der obengenannten Probleme wird durch ein Verfahren zur Herstellung eines Detektionssystems nach Anspruch 1, ein Detektionssystem nach Anspruch 10 und dessen Verwendung nach Anspruch 15 gelöst. Die abhängigen Ansprüche definieren weitere vorteilhafte Ausführungsformen der Gegenstände der unabhängigen Ansprüche.

Die vorliegende Erfindung bezieht sich auf ein Verfahren zur Herstellung eines Detektionssystems für Biomoleküle in einem Medium. Das Verfahren umfasst die Schritte:
- Bereitstellen eines ersten Detektorabschnittes mit einem ersten Kanalbereich und eines zweiten Detektorabschnittes mit einem zweiten Kanalbereich;
- Bereitstellen einer Membran mit zumindest einer Pore; und
- Anordnen des ersten Detektorabschnittes und des zweiten Detektorabschnittes auf gegenüberliegenden Seiten der Membranen, sodass zumindest ein Teil des ersten Kanalbereichs und des zweiten Kanalbereichs durch die Membranen getrennt werden und der erste Kanalbereich und der zweite Kanalbereich sich miteinander zu einem Kanalsystem verbinden, um einen Flusspfad für das Medium durch die zumindest eine Pore der Membran zu bilden,
wobei entlang des Flusspfades durch die Membranen Biorezeptoren an der Membran (z.B. im Porenbereich) ausgebildet sind, um eine Konzentration der Biomoleküle in dem Medium durch eine Messung des Flusses entlang der Flusspfades bestimmen zu können.

Der Begriff "Biomolekül" soll im Rahmen der vorliegenden Erfindung breit ausgelegt werden und insbesondere Liganden, Analyte, etc. umfassen. Das Medium kann jede beliebige Körperflüssigkeit sein (insbesondere Blut). Das System kann aber auch für die Wasseranalytik, Lebensmittelindustrie, Pharmaindustrie etc. verwendet werden, um bestimmte Stoffe zu detektieren. Die Membran kann einoder mehrteilig ausgebildet sein, sodass unter dem Begriff "Membran" ebenfalls verschiedene Membranen umfasst sein sollen. Ebenso soll der Begriff "Pore" breit ausgelegt werden und sich auf eine beliebige Öffnung oder einen beliebigen Kanal beziehen, solange die Öffnung/Kanal den Fluss erlaubt. Insbesondere soll die Pore nicht auf ein bestimmtes Aspektverhältnis (Länge-zu-Durchmesser) eingeschränkt werden.

Optional umfasst der Schritt des Anordnens Folgendes: Anordnen der Membran auf dem ersten Detektorabschnitt oder auf dem zweiten Detektorabschnitt; und danach Entfernen eines Teiles der Membran außerhalb eines Detektionsbereiches. Beispielsweise kann die Membran zunächst vollflächig auf einem der Detektorabschnitte aufgebracht werden und anschließend so strukturiert werden (z.B. zurechtgeschnitten werden), dass sie nur in einem Detektionsbereich zwischen dem ersten Kanalbereich und dem zweiten Kanalbereich angeordnet ist.

Optional umfasst das Verfahren weiter ein Ausbilden einer Klebeschicht, die in Kontakt zu der Membran steht. Die Klebeschicht kann derart in Kontakt zu der Membran gebracht werden, dass zumindest einige der Poren durch die Klebeschicht verschlossen werden, um dadurch eine Sensitivität der Membran durch eine Verringerung einer Anzahl von Poren für die Flussmessung des Mediums zu erhöhen. Beispielsweise kann die Klebeschicht genutzt werden, um gezielt einige Poren zu verschließen (zu zukleben).

Optional umfasst das Verfahren weiter ein Anbringen der Biorezeptoren an der Membran durch eine Funktionalisierung, wobei das Funktionalisieren vor oder nach dem Anordnen des ersten Detektorabschnittes und des zweiten Detektorabschnittes auf gegenüberliegenden Seiten der Membran ausgeführt wird. Es versteht sich, dass das Anbringen ebenfalls ein Ankoppeln und/oder Anbinden der Rezeptoren umfassen soll. Das Funktionalisieren kann beispielsweise zumindest die folgenden Funktionalisierungsschritte umfassen: Aktivieren einer Carboxy-Endgruppe, um ein Amin-reaktives Intermediat zu erhalten; und Amidisieren des Amin-reaktiven Intermediats, um gewünschte Biorezeptoren an der Membran auszubilden.

Dabei kann das Funktionalisieren in allen Bereichen der Membran gleich erfolgen. Es ist jedoch ebenfalls möglich, dass beim Funktionalisieren in verschiedenen Bereichen der Membran verschiedene Biorezeptoren in den Poren ausgebildet (oder angekoppelt oder angebunden) werden, sodass die Membran in verschiedenen Bereichen für verschiedene Biomoleküle sensitiv wird. Außerdem können die verschiedenen Funktionalisierungsschritte an einer einzigen Membran ausgeführt werden. Es ist jedoch ebenfalls möglich, dass die Membran mehrere Teile aufweist bzw. dass mehrere Membranen zur Detektion genutzt werden, die verschieden funktionalisiert werden sollen.

Optional umfasst das Verfahren weiter ein Laminieren der Membran auf den ersten Detektorabschnitt und/oder auf den zweiten Detektorabschnitt.

Der erste Detektorabschnitt und der zweite Detektorabschnitt können an den gegenüberliegenden Seiten der Membran durch eine thermische Behandlung bei einer Temperatur von zumindest 50°C oder zumindest 65°C miteinander verbunden werden. Dadurch kann eine ausreichende Dichtheit erreicht werden. Weiterhin ist es möglich, eine dichte Verbindung auch ohne eine TemperaturBehandlung zu erhalten, z.B. durch Verkleben.

Optional kann die Konzentration der Biomoleküle in dem Medium durch zumindest eine der folgenden Messungen bestimmt wird: (i) eine Flussmessung durch die zumindest eine Pore, (ii) eine Impedanzmessung, und (iii) eine elektrokinetische Messung, insbesondere eine Elektrophorese- oder eine Elektroosmose-Messung. Im einfachsten Fall kann eine elektrische Widerstandsmessung durchgeführt werden, die proportional zu dem Fluss des Mediums durch die Pore ist. Auf diese Weise kann eine Stromstärke und somit die Anzahl von Ladungsträgern (d.h. Ionen in Medium) gemessen werden, die pro Zeiteinheit die Pore passieren.

Optional umfassen die Biomoleküle ein Prostata-spezifisches Antigen (PSA) und die Biorezeptoren Aptamere. Die genutzten Aptamere können insbesondere eine der folgenden Aptamere sein:
a) 
NH₂-C₆-CCGUCAGGUCACGGCAGCGAAGCUCUAGGCGCG
GCCAGUUGC-OH;
b) NH₂-C₆-TTTTTAATTAAAGCTCGCCATCAAATAGCTTT-OH;
c)

NH₂-C₆-ACGCTCGGATGCCACTACAGGTTGGGGTCGGGCATGCGTC
CGGAGAAGGGCAAACGAGAGGTCACCAGCACGTCCATGAG-OH.

Die vorliegende Erfindung bezieht sich auch auf ein Detektionssystem für Biomoleküle in einem Medium. Das Detektionssystem umfasst Folgendes: einen ersten Kanalbereich und einen zweiten Kanalbereich, in die das Medium einbringbar ist, und eine Membran, die zumindest eine Pore aufweist und den ersten Kanalbereich von dem zweiten Kanalbereich trennt. Außerdem ist eine erste Elektrode und eine zweite Elektrode entlang einer Flußrichtung des Mediums auf gegenüberliegenden Seiten der Membran ausgebildet. An oder in der Pore sind Biorezeptoren ausgebildet oder angekoppelt oder angebunden, die zumindest eines der folgenden Aptamere umfassen:
(i) 
NH₂-C₆-CCGUCAGGUCACGGCAGCGAAGCUCUAGGCGCG
GCCAGUUGC-OH;
(ii) NH₂-C₆-TTTTTAATTAAAGCTCGCCATCAAATAGCTTT-OH;
(iii)

NH₂-C₆-ACGCTCGGATGCCACTACAGGTTGGGGTCGG
GCATGCGTCCGGAGAAGGGCAAACGAGAGGTCAC
CAGCACGTCCATGAG-OH.

Damit kann eine PSA-Konzentration im Medium über eine Widerstandsmessung entlang eines Flusspfades für das Medium zwischen der ersten Elektrode und der zweiten Elektrode gemessen werden. Im einfachsten Fall kann ein elektrischer Widerstand eines elektrolytischen Flusses (durch Anlegen einer Spannung zwischen den Elektroden) gemessen werden.

Die zumindest eine Pore in der Membran kann ein konisch zulaufendes oder ein zylindrisches Profil entlang des Flusspfades aufweisen.

Optional umfasst die Membran in verschiedenen Bereichen verschiedene Rezeptoren oder verschiedene Aptamere, um eine simultane Detektion verschiedener Biomoleküle zu ermöglichen.

Optional weist der erste Kanalbereich und/oder der zweite Kanalbereich senkrecht zum Flusspfad eine maximale Kanalbreite von 50 µm oder höchstens 10 µm auf. Dadurch wird es möglich, über eine Benetzung der Membran effektiv eine Ein-Porenmembran zu erreichen (z.B. wenn die Porendichte in der Membran entsprechend gewählt ist), wodurch sich die Sensitivität steigert. Die Kanalbreite kann aber auch bis zu 1 mm betragen. Eine Untergrenze liegt bei den verwendeten Materialien typischerweise bei 1 µm, sie könnte aber geringer werden sobald Silizium oder andere Materialien benutzt werden.

Optional umfasst das Detektionssystem weiter einen Elektrolyt-Einlass bei der zweiten Elektrode und einen Analyt-Einlass bei der ersten Elektrode, um das Medium in dem Analyt-Einlass und einen Elektrolyten in den Elektrolyt-Einlass einbringen zu können. Dadurch kann eine für die Detektion erforderliche Menge des Mediums verringert werden.

Die vorliegende Erfindung bezieht sich auch auf eine Verwendung oder ein Verfahren zur Verwendung eines der beschriebenen Detektionssysteme zur Detektion von Biomolekülen in einem Medium, wobei die Detektion durch ein Messen einer elektrischen Größe, die von einem elektrischen Widerstand zwischen der Elektrode und der zweiten Elektrode abhängt, durchgeführt wird.

Ausführungsbeispiele beziehen somit insbesondere auf einen elektrochemischen Sensor zur Detektion von biotischen und abiotischen Liganden (Biomoleküle). Dazu zählen unter anderem jede molekulare, organische und anorganische Verbindung jedweder Art, Umweltgifte, Agrarchemikalien, Hormone, Proteine, Antibiotika, Neurotoxine. Ebenso gehören dazu Bakterien, Viren und Parasiten, die Bestandteil von Organismengruppen sein können.

Ein Vorteil von Ausführungsbeispielen liegt darin, dass damit eine kostengünstige Alternative gegenüber dem Stand der Technik erreicht werden kann, die eine höhere Selektivität und Sensitivität aufweist. Die Erfindung ermöglicht weiter die Integration von Nanosensoren in einem mikrofluidischen System, welches als ein portables mobiles Analyt-System für diverse Anwendungen, wie sie beispielsweise oben genannt wurden, verwendet werden kann. Wegen der breiten Einsatzmöglichkeiten der Ausführungsbeispiele kann die vorliegende Erfindung insbesondere auch für Anwendungen genutzt werden, die bisher nicht oder apparativ nur sehr aufwendig analysiert werden konnten.

Außerdem besitzt die Funktionalisierung eine hohe Selektivität, so dass nur das PSA an der Pore angekoppelt/angebunden wird.

Insbesondere ermöglichen Ausführungsbeispiele, die Frühdiagnose von Prostatakrebs (PCa) signifikant zu vereinfachen und damit zu erleichtern.

### Kurzbeschreibung der Figuren

Die Ausführungsbeispiele der vorliegenden Erfindung werden besser verstanden anhand der folgenden detaillierten Beschreibung und den beiliegenden Zeichnungen der unterschiedlichen Ausführungsbeispiele, die jedoch nicht so verstanden werden sollten, dass sie die Offenbarung auf die spezifischen Ausführungsformen einschränken, sondern lediglich der Erklärung und dem Verständnis dienen.
- Fig.1: zeigt ein Flussdiagramm für ein Verfahren zur Herstellung eines Detektionssystems für Biomoleküle gemäß einem Ausführungsbeispiel der vorliegenden Erfindung.
- Fig. 2: veranschaulicht das zugrundeliegende Messprinzip unter Nutzung einer Membran mit zumindest einer Pore.
- Fig.3A,B: zeigen ein beispielhaftes Detektionssystem und die Messwerterfassung basierend auf dem Messprinzip aus der Fig. 2.
- Fig. 4: veranschaulicht eine beispielhafte Funktionalisierung der Membran.
- Fig. 5: zeigt ein Detektionssystem gemäß einem Ausführungsbeispiel der vorliegenden Erfindung.
- Fig. 6A-M: zeigen einen Prozessablauf zur Herstellung des Detektionssystems gemäß Ausführungsbeispielen.

- Fig. 7A,B: zeigen die mit dem Prozessablauf nach Fig. 6 fertiggestellten Detektionssysteme nach weiteren Ausführungsbeispielen der vorliegenden Erfindung.

### Detaillierte Beschreibung

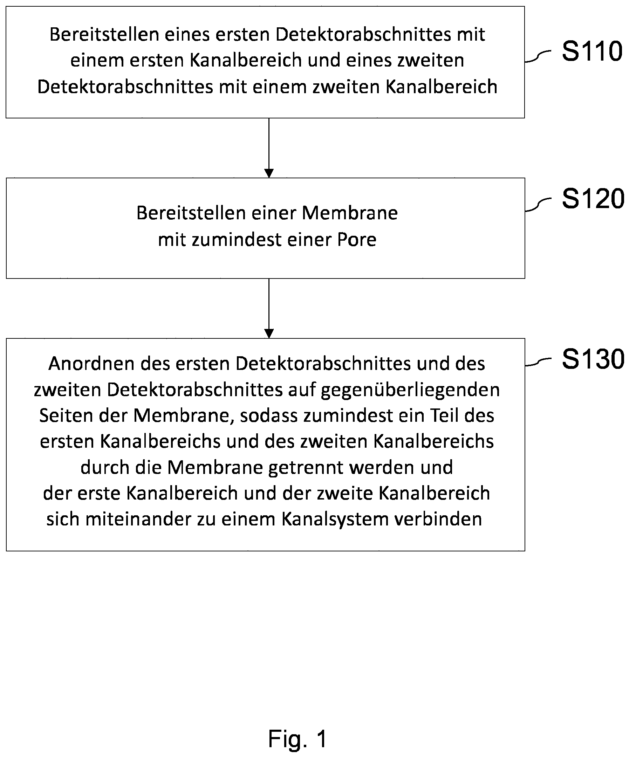

**Fig. 1** zeigt ein Flussdiagramm für ein Verfahren zur Herstellung eines Detektionssystems für Biomoleküle gemäß einem Ausführungsbeispiel der vorliegenden Erfindung. Das Verfahren umfasst:
- Bereitstellen S110 eines ersten Detektorabschnittes mit einem ersten Kanalbereich und eines zweiten Detektorabschnittes mit einem zweiten Kanalbereich;
- Bereitstellen S120 einer Membran mit zumindest einer Pore; und
- Anordnen S130 des ersten Detektorabschnittes und des zweiten Detektorabschnittes auf gegenüberliegenden Seiten der Membran, sodass zumindest ein Teil des ersten Kanalbereichs und des zweiten Kanalbereichs durch die Membran getrennt werden und der erste Kanalbereich und der zweite Kanalbereich sich miteinander zu einem Kanalsystem verbinden, um einen Flusspfad für das Medium durch die zumindest eine Pore der Membrane zu bilden.

Es versteht sich, dass diese Aufzählung keine Reihenfolge impliziert. Die genannten Herstellungsschritte können separat unabhängig voneinander bzw. parallel durchgeführt werden. Entlang des Flusspfades sind durch die Membran-Biorezeptoren an der Membran ausgebildet, um eine Konzentration der Biomoleküle in dem Medium durch eine Messung des Flusses (z.B. des Widerstandes) entlang der Flusspfades zu bestimmen.

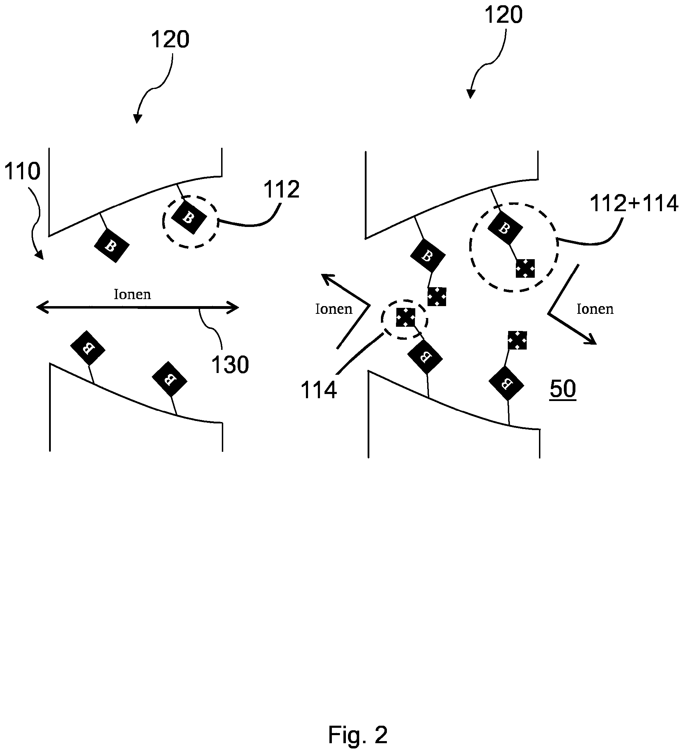

**Fig. 2** veranschaulicht das zugrundeliegende Messprinzip unter Nutzung der Membran 120 mit der zumindest einen Pore 110. Diese Pore 110 ist z.B. ein Nanokanal mit einem (maximalen) Durchmesser von weniger als 1 µm (kann beispielsweise nur einigen Nanometer oder weniger als 100 nm sein). Die Membran 120 ist beispielsweise eine Einzelpore-Kunststofffolie.

Auf der linken Seite der Fig. 2 ist eine Querschnittsdarstellung durch die Pore 110 gezeigt, wobei innerhalb der Pore 110 Biorezeptoren 112 angebracht oder ausgebildet sind. Diese Biorezeptoren 112 sind derart beschaffen, dass die zu detektierenden Moleküle 114 (Biomolekül oder Analyt-Moleküle) beim Ausbilden eines Ionenstroms 130 durch die Membran 120 daran haften bleiben oder daran gebunden werden (siehe rechte Seite auf der Fig. 2). Der Ionenstrom 130 kann beispielsweise durch ein elektrisches Feld erzeugt werden, das auf geladenen Ionen in dem Ionenstrom 130 wirkt. Durch das Ankoppeln und/oder Anbinden der Biomoleküle 114 an den Biorezeptoren 112 ändert sich der Widerstand für den Ionenstrom 130 durch die Pore 110. Diese Änderung kann über eine elektrische Messung gemessen werden.

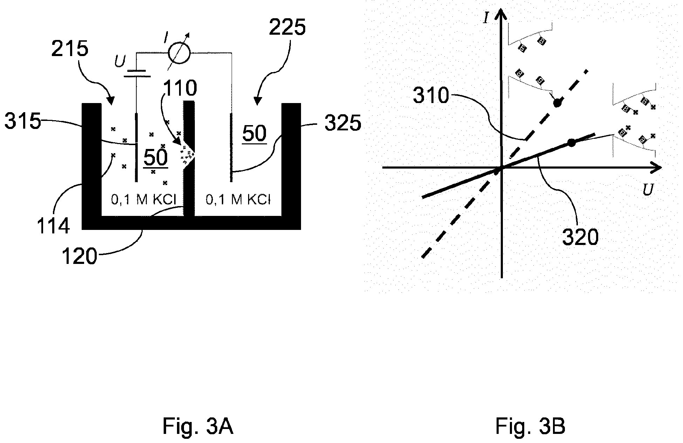

**Fig.3A** zeigt ein beispielhaftes Detektionssystem basierend auf dem beschriebenen Messprinzip aus der Fig.2, wobei beispielhaft der Aufbau einer elektrochemischen Messzelle mit einer Einzelporen-Kunststofffolie 120 gezeigt ist. Eine damit messbare Stromspannungscharakteristik (*I*/*U-Kennlinie*) ist in der **Fig. 3B** gezeigt, wobei die Änderung der Kennlinie durch die Analyt-Konzentration in dem Medium 50 verursacht wird. Die Bindung von Analyt/Liganden-Molekülen 112 +114 an der funktionalisierten Nanopore 110 ist daher mit einer (qualitativen) *I*/*U*-Messung ermittelbar.

Das Detektionssystem umfasst im Detail einen ersten Kanalbereich 215 und einen zweiten Kanalbereich 225 mit der dazwischen angeordneten Membran 120 (siehe Fig. 3A). Auch wenn die Erfindung darauf nicht eingeschränkt werden soll, versteht es sich, dass der erste und zweite Kanalbereich 215, 225 typischerweise Teile eines Kanalsystems darstellen, durch welches das Medium 50 sich bewegt. Das Medium kann beispielsweise ein Elektrolyt (z.B. 0,1 M KCl, M=mol/L) mit oder ohne Biomolekülen sein. Der Messaufbau kann auch einen Elektrolyt-Behälter aufweisen, der durch die Membran 120 in zwei Hälften 215, 225 geteilt ist. Die Membran 120 kann einen oder mehrere Nanokanäle 110 als Poren enthalten, die auf der Oberfläche mit kovalent gebundenen Rezeptormolekülen 112 derivatisiert sein können. Die kovalent gebundenen Rezeptormolekülen 112 können optional (nahezu) überall auf der Membran 120 vorhanden sein. Die Rezeptoren 112 sind in der Lage, die Biomoleküle (Analyte, Liganden) 114 selektiv und hochaffin zu binden, wodurch der elektrische Widerstand der Nanokanäle 110 in Abhängigkeit zur Konzentration der Liganden-Moleküle 114 steigt.

In dem Medium 50 befinden sich Ionen (z.B. als Teil des Elektrolyten) und die zu detektierenden Biomoleküle 114, die ebenfalls Ionen sein können (müssen es aber nicht). In dem ersten Kanalbereich des 215 befindet sich außerdem eine erste Elektrode 315 und in den zweiten Kanalbereich 225 befindet sich eine zweite Elektrode 325. Durch das Anlegen der Spannung U zwischen der ersten Elektrode 315 und der zweiten Elektroden 325 fließt ein Strom *I* durch den Nanokanal 110 (siehe Fig. 2). Der Strom *I* führt dazu, dass die Biomoleküle 114 an den Rezeptormolekülen 112 in der Pore 110 haften bleiben und wie gesagt einen elektrischen Widerstand in Abhängigkeit von einer Menge der vorhandenen Biomoleküle 114 ändern. Je mehr Biomoleküle 114 vorhanden sind, umso mehr bleiben potentiell in der Pore 110 haften und verringern somit deren Querschnitt, der dem Ionenstrom 130 zur Verfügung steht.

Die Änderung des elektrischen Widerstandes kann durch eine Stromspannungsmessung ermittelt werden. Die entsprechende Charakteristik ist in der Fig. 3B dargestellt. Beispielhaft sind zwei Kennlinien gezeigt. Eine erste Kennlinie 310 zeigt einen gemessenen Strom *I* in Abhängigkeit der angelegten Spannung *U*, wenn keine Biomoleküle 114 in dem Medium 50 vorhanden sind. Die zweite Kennlinie 320 zeigt die Strom-Spannungsabhängigkeit für den Fall, dass eine größere Anzahl von Biomolekülen 114 in dem Medium 50 vorhanden ist. Wie dargestellt verringert sich als Folge der Biomoleküle 114 der Strom *I* für eine gegebene Spannung *U*, was eine Folge des erhöhten Widerstandes beim Passieren der Pore 110 ist.

Wie eingangs erwähnt, ist eine entsprechende Funktionalisierung der Membran erforderlich, bei der innerhalb der Pore 110 entsprechende Biorezeptoren 112 anbringt werden, sodass eine hohe Sensitivität der Membran für bestimmte zu detektierende Moleküle erreicht wird. Auch die Pore(n) selbst kann/können während der Funktionalisierung erzeugt werden.

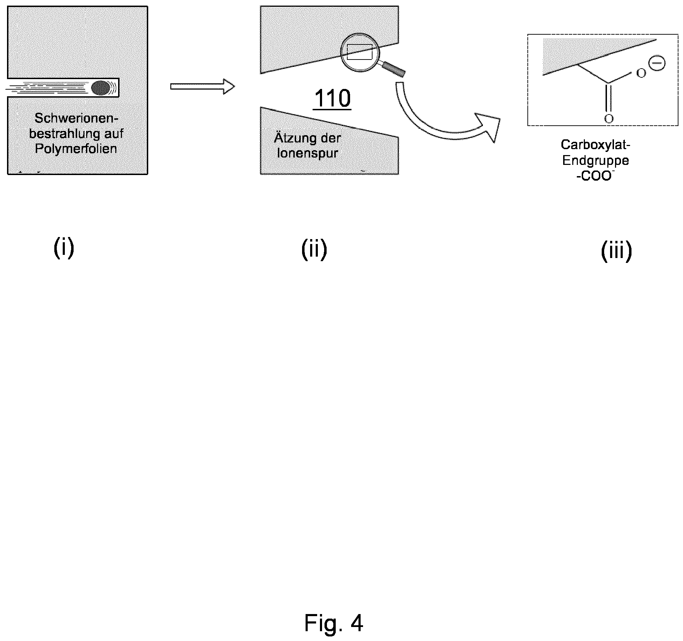

**Fig. 4** veranschaulicht eine beispielhafte Funktionalisierung. Zunächst erfolgt eine Generierung der Carbonsäure/Caboxylat-Endgruppen auf der Porenoberfläche durch einen Bestrahlungs- und einen Ätzprozess. Dies kann beispielsweise in den gezeigten drei Schritten (i) - (iii) durchgeführt werden. Im ersten Schritt (i) wird die Membran 120 beispielsweise mit Schwerionen bestrahlt, sodass die Ionen in die Membran 120 eintreten und die gesamte Membran 120 durchdringen können und so eine Öffnung erzeugen oder zumindest die chemischen Bindungen dort aufbrechen. Als zweiter Schritt (ii) erfolgt ein Ätzschritt, der dazu führt, dass die Ionenspur verbreitert wird und sich eine konisch zulaufende Pore 110 ergibt. Schließlich kann auf der Oberfläche der Pore 110 eine Carboxylat-Endgruppe ausgebildet werden, die sensitiv für die zu detektierenden Moleküle 114 ist bzw. die als Ankerpunkt für eine Anbringung der Rezeptoren der zu detektierenden Moleküle 114 dienen kann/dient.

Die Oberflächeneigenschaften sind durch eine kovalente Verknüpfung mit unterschiedlichen Rezeptormolekülen 112 wie zum Beispiel Nukleinsäure-Aptameren (DNA/RNA) einstellbar. Nach Ali et al. (Ali M, Nasir S, Ensinger W. 2015. Bioconjugation-induced ionic current rectification in aptamer-modified single cylindrical nanopores. Chem Commun 51: 3454-3459) kann die Kopplung in einer Zweischrittreaktion unter Verwendung von 1-Ethyl-3-(3-dimethylaminopropyl)carbodiimid (EDC) und Sulfo-NHS (N-hydroxysulfosuccinimid) erfolgen. Der Reaktionsmechanismus der Verknüpfung eines biologischen Rezeptors 112 (z.B. eines Aptamers) mit an der Oberfläche lokalisierten Carbonsäure/Carboxylat-Gruppen durch EDC/NHS-Kopplungschemie kann wie folgt dargestellt werden:

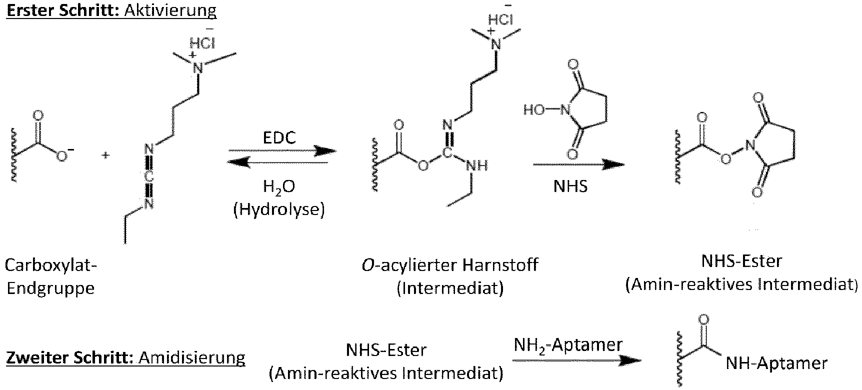

Gemäß Ausführungsbeispielen der vorliegenden Erfindung findet die Reaktion in dem mikrofluidischen System statt, dessen Aufbau und Fertigung weiter unten näher erläutert wird.

Erster Schritt (Aktivierung): Hier werden die Carboxy-Endgruppen durch die Veresterung von NHS mittels EDC aktiviert. Es entsteht zunächst ein O-acyliertes Harnstoff-Zwischenprodukt, welches in einen Amin-reaktiven NHS-Ester umgewandelt wird. Hierfür wird die Membran 120 in das mikrofluidische System integriert. Anschließend wird das System mit einer frisch hergestellten wässrigen Lösung (pH 7) von 0,2 mM EDC und 0,4 mM NHS befüllt. Nach einer Stunde ist die Aktivierung der Oberfläche der Poren 110 abgeschlossen.

Zweiter Schritt (Amidisierung): Hier erfolgt die Funktionalisierung mit den Rezeptormolekülen 112 (Aptameren), deren chemische Struktur mindestens eine primäre Aminogruppe (-NH2) enthält. Diese Aminogruppe reagiert bei Raumtemperatur mit dem aktivierten Carbonsäure-Ester unter Ausbildung einer Amid-Bindung (-(C=O)-NH-). Hierzu wird das mikrofluidische System mit einer 0,1 mM wässrigen Lösung des Rezeptormoleküls 112 (Aptamers) befüllt und über Nacht stehen gelassen.

Die erfolgreiche Funktionalisierung wird über die Messung einer Strom-Spannungs-Kennlinie verifiziert, da unfunktionalisierte und funktionalisierte Poren 110 sich bei gleichem Potential durch unterschiedliche Stromstärken unterscheiden. Dieses sensorische Prinzip wurde bereits mit den Fig. 2 und 3 erläutert.

Als PSA-spezifische Aptamere als Biorezeptoren 112 sollen nachfolgende Moleküle Verwendung finden sollen:
1. RNA Aptamer (Referenz: Jeong S, Han SR, Lee YJ, Lee SW. 2010. Selection of RNA aptamers specific to active prostate-specific antigen. Biotechnol Lett 32:379-385) Sequenz (5'-3'):
   NH₂-C₆-CCGUCAGGUCACGGCAGCGAAGCUCUAGGCGCGGCCAGUUGC-OH
2. DNA Aptamer-01 (Referenz: Savory N, Abe K, Sode K, Ikebukuro K. 2010. Selection of DNA aptamer against prostate specific antigen using a genetic algorithm and application to sensing. Biosens Bioelectron 26:1386-1391) Sequenz (5'-3'):
   NH₂-C₆-TTTTTAATTAAAGCTCGCCATCAAATAGCTTT-OH
3. DNA Aptamer-02 (Referenz: Duan M, Long Y, Yang C, Wu X, Sun Y, Li J, Hu X, Lin W, Han D, Zhao Y, Liu J, Ye M, Tan W. 2016. Selection and characterization of DNA aptamer for metastatic prostate cancer recognition and tissue imaging. Oncotarget 7:36436-36446) Sequenz (5'-3'):

NH₂-C₆-ACGCTCGGATGCCACTACAGGTTGGGGTCGG
GCATGCGTCCGGAGAAGGGCAAACGAGAGGTCACCAGCACGTCCATGAG-OH

Die sensorischen Eigenschaften der funktionalisierten (Einzelporen-) Kunststofffolien 120 können in einer Makrozelle untersucht werden. Hierzu kann die beispielhafte Einzelporen-Kunststofffolien 120 genutzt werden, die vor jeder Untersuchung manuell zwischen zwei Flüssigkeitskammern 215, 225 eingespannt werden. Die vorteilhaften Einzelporen-Kunststofffolien 120 sind nur aufwendig herzustellen. Im Gegensatz dazu können Multiporen-Kunststofffolien als Massenprodukte gefertigt werden. Allerdings haben sie eine geringere Sensitivität gegenüber den Einzelporen.

Um die Vorteile beider Folien zu kombinieren, wird gemäß Ausführungsbeispielen die Benetzungsfläche der Multiporen-Folie 120 so weit verkleinert, dass eine einzelne Pore noch Kontakt mit der Flüssigkeit hat. Diese erfolgt durch die Integration in ein Mikrosystem und ermöglicht somit die Verwendung des Detektionssystems von ungeschulten Anwendern.

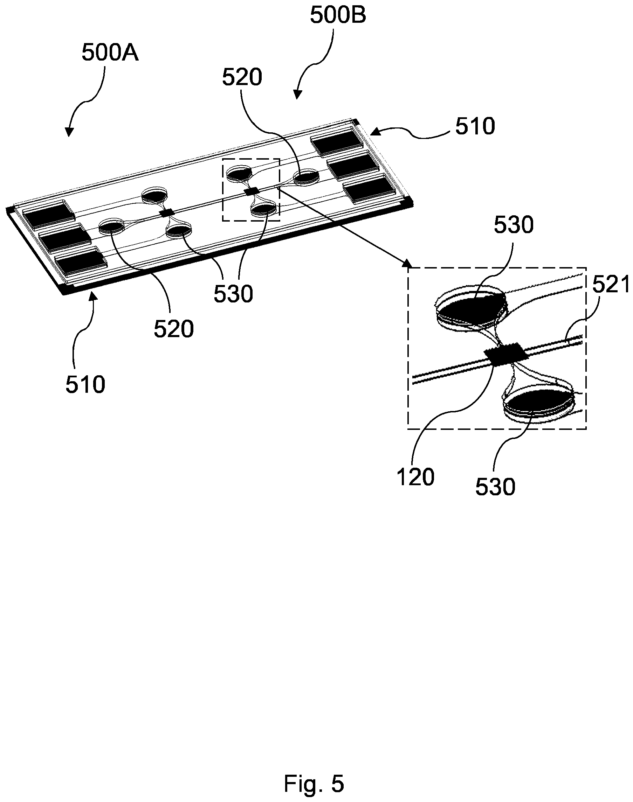

**Fig. 5** zeigt beispielhaft ein mögliches Detektionssystem, welches zwei Detektorbereiche 500A und 500B umfasst, die für unterschiedliche Analysen (Detektion verschiedener Biomoleküle) genutzt werden können. Jeder der zwei Detektorbereiche 500A, 500B umfasst drei Anschlusselektroden 510, einen Einlass 520 für einen Elektrolyten und jeweils zwei Einlässe 530 für einen Analyten. Der Analyt ist beispielsweise das zu untersuchende Medium 50 mit den Biomolekülen 114 (Analyte) und der Elektrolyt kann jede Flüssigkeit sein, die Ionen enthält (um den elektrischen Strom zu unterstützen) und das Ergebnis nicht verfälscht.

In dem Mikrofluidik-System aus der Fig. 5 ist zwischen den Kanälen für die Elektrolyt-Moleküle und für die Biomoleküle 114 eine Multiporen-Kunststofffolie 120 integriert. Alle Einzelteile werden durch eine Klebeschicht zusammengefügt. Das Mikrofluidik-System kann in ein elektronisches Messsystem in Form eines Tischgeräts integriert werden.

Auf der rechten Seite der Fig. 5 ist der zweite Detektorbereich 500B vergrößert dargestellt, wobei die Membran 120 zwischen zwei Analyt-Einlässen 530 ausgebildet ist und ein Kanal 521 zu dem betreffenden Elektrolyt-Einlass 520 führt. Es versteht sich, dass alle Einlässe auch Auslässe sein können. Dazu braucht lediglich die Strömungsrichtung umgedreht werden. Daher können die Analyt-Einlässe 530 und Elektrolyt-Einlass 520 auch entsprechende Auslässe darstellen. Die Erfindung soll nicht auf eine bestimmte Strömungsrichtung eingeschränkt werden. Beispielsweise besteht eine fluide Verbindung zwischen den beiden Analyt-Einlässen 530 zu einer Seite der Membran 120. Die gegenüberliegende Seite der Membran 120 kann beispielsweise fluid mit dem Elektrolyt-Einlass 520 verbunden sein. Außerdem sind in den Analyt-Einlässen 530 Elektroden ausgebildet, die mit jeweils einer der Anschlusselektroden 510 verbunden sind. Ebenso ist in dem Elektrolyt-Einlass 520 eine Elektrode ausgebildet, die mit einer der Anschlusselektroden 510 verbunden ist. Durch ein gezieltes Anlegen einer Spannung zwischen der Elektrode in dem Elektrolyt-Einlass 520 und einem der Elektroden in den Analytik-Einlässen 530 wird ein Fluss des Analyts 50+114 von dem jeweiligen Einlass 530 hin zu dem Elektrolyteinlass 520 erzeugt.

Die Membran 120 ist dabei beispielhaft horizontal ausgebildet und der Analytfluss von einem der Analyt-Einlässe 530 erfolgt beispielsweise in vertikaler Richtung durch die Membran 120 hindurch hin zu dem Kanal 521, der zu dem Elektrolyt-Einlass 520 führt. Dieser Fluss kann entweder vertikal nach unten oder vertikal nach oben durch eine angelegte Spannung an den entsprechenden Elektroden erzeugt werden. Die Funktionsweise des Detektionssystems wird nachfolgend über die Darstellung der Herstellung weiter veranschaulicht. Da mehrere Analyt-Einlässe 530 vorhanden sind, können parallel oder nacheinander verschiedene Messungen durchgeführt werden (z.B. für verschiedene Biomoleküle 114). So können die verschiedenen Analyt-Einlässe 530 zu verschiedenen Bereichen der Membran 120 geführt werden, die unterschiedlich funktionalisiert sind, um so eine Analyse für verschiedene Biomoleküle 114 parallel zu erlauben.

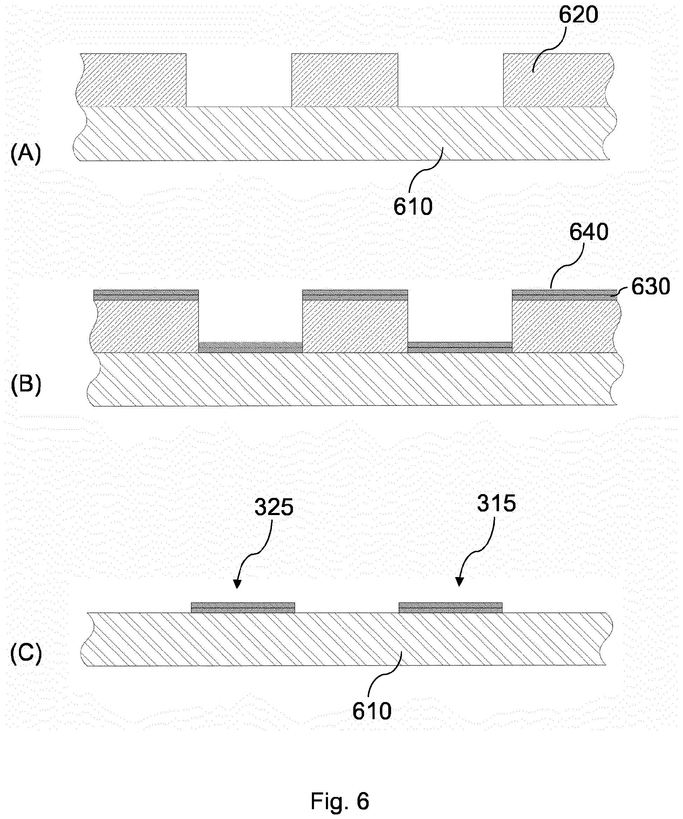

**Fig.6A bis 6M** veranschaulichen die verschiedenen Schritte bei der Herstellung des beispielhaften Detektionssystems, wie es zum Beispiel in der Fig. 5 dargestellt ist.

Die Fig. 6A bis 6C veranschaulichen zunächst die Herstellung der Elektroden. Dazu wird auf einem Substrat 610 (zum Beispiel einem Glassubstrat) abschnittsweise eine Photoresistschicht 620 aufgebracht. Im Anschluss wird auf die Photoresistschicht 620 und den freiliegenden Glassubstratabschnitten 610 eine Zwischenschicht (z.B. eine Chromschicht) 630 und eine Elektrodenschicht 640 (z.B. eine Silberschicht) abgeschieden. Das Abscheiden kann beispielsweise durch eine physikalische Gasphasenabscheidung (PVD), z.B. durch Sputtern oder Aufdampfen, geschehen. Schließlich wird die Photoresistschicht 620 zusammen mit den darauf ausgebildeten beispielhaften Chrom- und Silberschichten entfernt, sodass, wie in der Fig. 6C gezeigt, das Glassubstrat 610 mit strukturierten Elektroden 315, 325 entsteht. Die Elektroden können beispielsweise die erste Elektrode 315 und/oder die zweite Elektrode 325 darstellen.

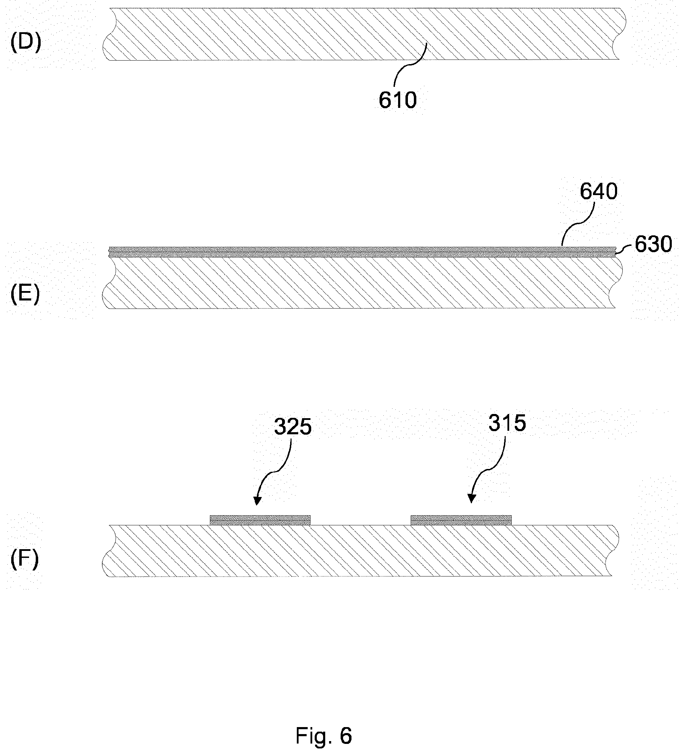

Die Fig. 6D bis 6F veranschaulichen eine weitere Möglichkeit, um Elektroden auszubilden. Auch hier wird auf einem Substrat 610 zunächst eine beispielhafte Zwischenschicht 630 (z.B. eine Chromschicht) und darauf eine Elektrodenschicht 640 aufgebracht, die in diesem Ausführungsbeispiel Gold aufweisen kann. Das Aufbringen der Schichten auf dem Substrat 610 kann flächenweise erfolgen. Anschließend wird eine Strukturierung vorgenommen, d.h. die beispielhaften Gold- und Chromschichten 630, 640 werden in verschiedenen Bereichen entfernt, sodass wiederum lediglich die Elektroden 315, 325 übrigbleiben.

Die so erzeugte Elektrodenstruktur ist ebenfalls in der Fig. 5 zu sehen, wo die verschiedenen Anschlusselektroden 510 mit Elektroden in den entsprechenden Einlässen 520, 530 für den Elektrolyten und den Analyten verbunden sind. Die Elektroden können wiederum die erste Elektrode 315 und/oder die zweite Elektrode 325 darstellen.

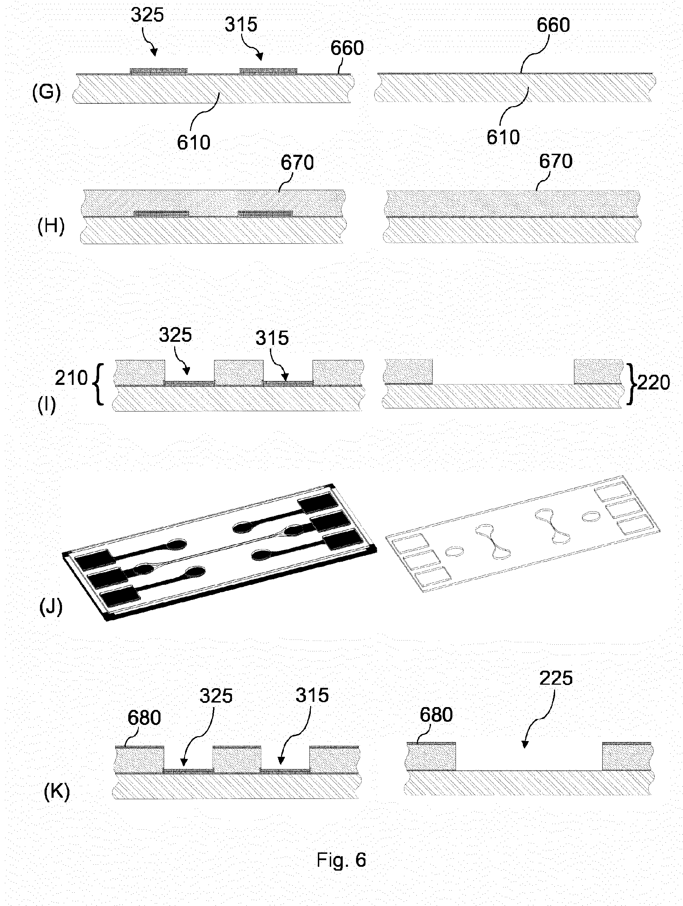

Die Fig. 6G bis 6M veranschaulichen die Herstellung des Detektionssystems. Gemäß Ausführungsbeispielen wird dabei ein erster Detektorabschnitt 210 und ein zweiter Detektorabschnitt 220 erzeugt (siehe Fig.6I), die daran anschließend aufeinander gebracht werden, um so das Detektionssystem zu bilden. Dazu werden in dem ersten Detektorabschnitt 210 und in dem zweiten Detektorabschnitt 220 Kanalstrukturen ausgebildet, die schließlich die Kanäle für den Analyten und/oder den Elektrolyten darstellen.

In der Fig. 6G ist zunächst auf der linken Seite beispielhaft das Substrat 610 mit der darauf ausgebildeten ersten Elektrode 315 und zweiten Elektrode 325 zu sehen, wie sie mit den Schritten aus den Fign. 6A bis 6F hergestellt werden können. Dies kann später der erste Detektorabschnitt 210 werden. Auf der rechten Seite wird in der Fig. 6G der zweite Detektorabschnitt 220 gefertigt, wobei wiederum zunächst das Substrat 610 dargestellt ist. Auf die dargestellten Abschnitte wird als nächstes eine Haftmittelschicht 660 ausgebildet. Die Haftmittelschicht 660 kann beispielsweise ein Titanmaterial aufweisen und eine beispielhafte Dicke von 0,5 µm umfassen.

In dem Herstellungsschritt aus der Fig. 6H wird auf den Strukturen aus der Fig. 6G eine Maskenschicht 670 (z.B. Trockenresist aus Epoxid) aufgebracht.

In dem folgenden Herstellungsschritt aus der Fig. 6I wird die Maskenschicht 670 strukturiert, wobei an den Stellen der Elektroden 315, 325 in dem ersten Detektorabschnitt 210 und in einem zentralen Bereich des zweiten Detektorabschnittes 220 die Maskenschicht 670 entfernt wird. Als Resultat werden die erste Elektrode 315 und die zweite Elektrode 325 in dem ersten Detektorabschnitt 210 und das Substrat 610 in dem zweiten Detektorabschnitt 220 freigelegt. Die Eigenschaften der resultierenden Kanäle (insbesondere die Hydrophilität) können durch eine Beschichtung modifiziert werden

Das Ergebnis ist in der Raumdarstellung der Fig. 6J dargestellt. Somit ist in dem ersten Detektorabschnitt 210 eine Vielzahl von Elektroden ausgebildet, wie sie beispielsweise in der Fig. 5 dargestellt sind.

In der Fig. 6K wird beispielhaft eine Klebeschicht 680 (z.B. eine Haftmittel- oder Laminatschicht insbesondere eine Trocken-Epoxid-Laminatschicht) auf die Strukturen aus der Fig. 6I aufgebracht.

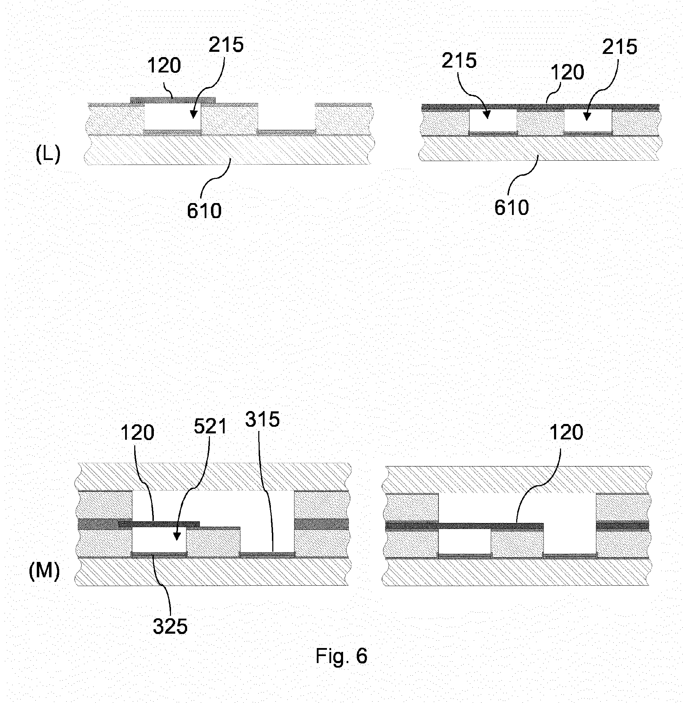

In dem folgenden Schritt (siehe Fig. 6L) wird die Membran 120 auf die hergestellten Strukturen aus der Fig. 6K aufgebracht. Gemäß Ausführungsbeispielen kann die Membran 120 beispielsweise vollflächig aufgebracht und thermisch laminiert werden (bei ca. T=65 °C); siehe rechte Seite der Fig. 6L. Sie braucht aber auch nur teilweise aufgebracht zu werden (siehe linke Seite der Fig. 6L), wobei die Mutliporen-Membran 120 zwischen die Kanäle oder Kanalbereiche positioniert und anschließend thermisch laminiert wird.

Falls die Membran 120 vollflächig auf die Strukturen der Fig. 6K aufgebracht wird (siehe rechte Seite von Fig.6L), erfolgt anschließend eine Strukturierung oder ein Entfernen der Membran 120, zum Beispiel an jenen Abschnitten, die anschließend eine Verbindung zwischen den Kanalbereichen (z.B. der erste und zweite Kanalbereich 215, 225) der erste Detektorabschnitt 210 und der zweite Detektorabschnitt 220 bilden sollen (siehe Fig. 6M). Abschließend wird der erste Detektorabschnitt 210 und der zweite Detektorabschnitt 220 aufeinander gesetzt, sodass die (strukturierte) Membran 120 zwischen dem ersten Detektorabschnitt 210 und dem zweiten Detektorabschnitt 220 angeordnet ist. Schließlich kann ein Laminieren des hergestellten Detektors erfolgen, um alle Schichten miteinander zu verbinden und dicht zu verschließen.

Die in der Fig. 6M gezeigten Kanalbereiche 215, 225 stellen beispielsweise die fluide Verbindung zwischen dem Analyt-Einlass 530, durch die Membran 120, über den Kanal 521 hin zu dem Elektrolyt-Einlass 520 dar (siehe Fig. 5). Die erste Elektrode 315 ist beispielsweise unterhalb dem Analyt-Einlasses 530 in der Fig. 5 ausgebildet, und die zweite Elektrode 325 ist beispielsweise die mittlere Elektrode, die über den Kanal 521 hin zu dem Elektrolyt-Einlass 520 geführt ist.

Gemäß Ausführungsbeispielen erfolgt abschließend (z.B. während des Herstellungsschrittes aus der Fig. 6M) die zuvor beschriebene Funktionalisierung der Membran 120.

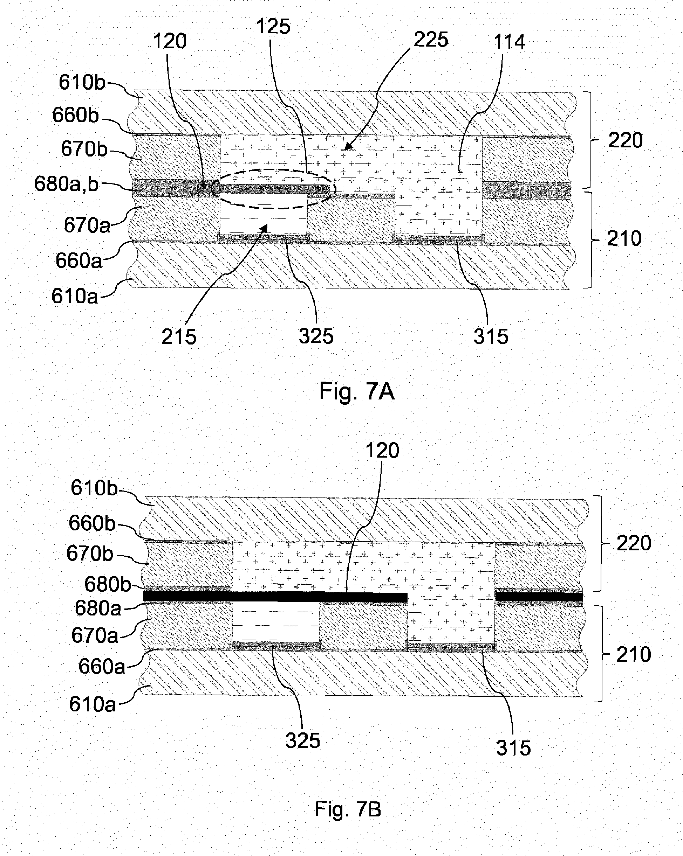

**Fig. 7A****,B** zeigen fertiggestellte Detektionssysteme nach Ausführungsbeispielen der vorliegenden Erfindung.

Das Ausführungsbeispiel der Fig. 7A zeigt ein Detektionssystem, bei dem zwischen dem ersten Detektorabschnitt 210 und dem zweiten Detektorabschnitt 220 die Membran 120 ausgebildet ist, und zwar im Wesentlichen (z.B. zu mehr als 50% oder zu mehr als 80%) nur in einem Detektionsbereich 125, wo sie den ersten Kanalbereich 215 und den zweiten Kanalbereich 225 trennt (bis auf Auflageflächen zur Fixierung).

Wie mit der Fig. 6 beschrieben, umfassen sowohl der erste Detektorabschnitt 210 als auch der zweite Detektorabschnitt 220 jeweils ein Substrat 610a, 610b, zwischen denen alle weiteren Schichten ausgebildet sind. Der zweite Detektorabschnitt 220 kann auch ohne Substrat hergestellt werden. Beginnend mit dem ersten Detektorabschnitt 210 ist auf dem entsprechenden Substrat 610a (siehe Fign. 6G-6I) zunächst eine Haftmittelschicht 660a ausgebildet, darauf ist eine Maskenschicht 670a ausgebildet und darauf ist eine Klebeschicht 680a ausgebildet. Unterhalb des Substrates 610b des zweiten Detektorabschnittes 220 ist wiederum zunächst eine Haftmittelschicht 660b aufgebracht, darunter eine Maskenschicht 670b, auf die wiederum die Klebeschicht 680b aufgebracht.

Außerdem sind auf dem Substrat 610a des ersten Detektorabschnittes 210 die erste Elektrode 315 und die zweite Elektrode 325 ausgebildet (siehe Fig. 6A-C). Dementsprechend ist zwischen der ersten Elektrode 315 und der zweiten Elektrode 325 ein Flusspfad 130 durch die Membran 120 ausgebildet, der einen Strom beim Anlegen einer Spannung zwischen der ersten Elektrode 315 und der zweiten Elektrode 325 auslöst, dessen Widerstand durch die Pore (in der Fig. 7A nicht gezeigt) gemessen werden kann und zur Feststellung der Konzentration der Biomoleküle 114 in dem Medium 50 genutzt werden kann.

Das Ausführungsbeispiel der Fig. 7B unterscheidet sich von dem Ausführungsbeispiel der Fig. 7A lediglich dadurch, dass die Membran 120 beim Anordnen zwischen dem ersten Detektorabschnitt 210 und dem zweiten Detektorabschnitt 220 (im Wesentlichen) nur an der Stelle entfernt wurde, wo der Flusspfad 130 ausgehend von der ersten Elektrode 315 hin zu der Membran 120 den ersten Detektorabschnitt 210 verlässt und in den zweiten Detektorabschnitt 220 eintritt. Ansonsten ist die Membran 120 insbesondere zwischen den Klebeschichten 680a,b, die als Teil des ersten Detektorabschnittes 210 und des zweiten Detektorabschnittes 220 ausgebildet wurde, immer noch vorhanden.

Somit ist in dem Ausführungsbeispiel der Fig. 7B die erste Klebeschicht 680a von der zweiten Klebeschicht 680b durch die Membran 120 zumindest teilweise oder überwiegend getrennt - insbesondere auch außerhalb des Detektionsbereiches 125.

Vorteilhafte Aspekte von Ausführungsbeispielen der vorliegenden Erfindung beziehen sich insbesondere auf das Folgende:
- Die groß-/ganzflächige Poren-Kunststofffolie 120 wird in ein *Lab-on-Chip*-System zwischen zwei Fluidkanälen 215, 225 integriert (z.B. in einem *Batch*-Verfahren).
- Die Folie 120 wird im Bereich der Fluidkanäle 215, 225 durch Laserschneiden, Xurography oder Ätzen entfernt (siehe Fig. 6L, 6M rechts).
- Die verwendete Porenfolie 120 umfasst konische Poren 110, mit reproduzierbarer Geometrie.
- Die Klebeschicht 680 dient sowohl der Integration der Pore 110, als auch zu ihrer Dysfunktionalisierung (Schließen) der Poren. Somit kann statistisch gesehen jeweils nur eine Pore in Kontakt mit dem Elektrolyten vorhanden sein. So wird die hohe Sensitivität von Einzelporen-Kunststofffolien 120 mithilfe von Multiporen-Kunststofffolien erreicht.
- Die Funktionalisierung der Poren 110 erfolgt nach der Chipherstellung, kann aber auch vor der Funktionalisierung erfolgen.

Die Funktionalisierung nach der Chipherstellung hat gegenüber den vorfunktionalisierten Poren folgende Vorteile:
- Nur geringe Mengen an Rezeptormolekülen 112 wird für die Funktionalisierung der Multiporen-Kunststofffolien 120 nach der Integration benötigt.
- Durch die Integration vorab funktionalisierter Poren besteht die Möglichkeit Poren zu kontaminieren bzw. zu verstopfen.

Ausführungsbeispiele bieten außerdem die folgenden Vorteile:
- Das neue System hat das Potential auf ein *Micro Total Analysis System* (µTAS) erweitert zu werden. Dies ermöglicht die simultane Detektion mehrerer Ligandenmoleküle 114.
- Die Sensitivität des Mikrosystems ist vergleichbar zu den Einzelporen-Messungen.
- Eine konventionelle Kleberschicht ist ein flüssiger UV-Kleber. Dieser führt zur Verstopfung der Poren und ist deshalb für die Integration von Poren bzw. funktionalisierten Poren nicht geeignet. Mit Hilfe dieser konventionellen Verfahren kann die Dichtigkeit des Systems nicht gesichert werden. Außerdem kann die Funktionalisierung der Folie durch die UV-Belichtung zerstört werden. Im Gegensatz hierzu wird bei Ausführungsbeispielen der Erfindung die Multiporen-Kunststofffolie 120 thermisch integriert (bei T=65 °C).
- Die verwendeten Multiporen-Kunststofffolien können sowohl nach, als auch vor der Integration funktionalisiert werden. Mit dieser Methode wurde eine Ausbeute von 100 % erreicht.

Eine Kanalbereite von 50 µm kann genutzt werden, was einer Benetzungsfläche von 2.500 µm² entspricht. Die Benetzungsfläche kann auf 100 µm² weiter reduziert werden. In konventionellen Verfahren ist nur eine Benetzungsfläche von 31.416 µm² erreicht worden.

Das bisher beschriebene Funktionsprinzip basiert auf einem voltametrischen Verfahren. Bei weiteren Ausführungsbeispielen werden andere Messprinzipien genutzt. Diese sind zum Beispiel:
(i) Fluss-Messung durch die Pore 110;
(ii) Impedanz-Messungen; und
(iii) Elektrokinetische Messungen (Elektrophorese, Elektroosmose, etc.).

Aber auch diese Messprinzipien messen letztlich einen Widerstand, der den Fluss der Biomoleküle 114 durch die Pore 110 behindert. Es ändert sich lediglich die erfasste Messgröße: in (i) die Flussgeschwindigkeit des Mediums 50; in (ii) eine elektrische Impedanz; in (iii) eine elektrokinematische Größe.

Im Vergleich zu gegenwärtigen Verfahren, die aufwendig die jeweiligen Analyt-/Ligandenmoleküle detektieren, ermöglichen Ausführungsbeispiele der vorliegenden Erfindung eine Konzentrationsmessung mit höherer Selektivität und Sensitivität gegenüber den derzeit vorhandenen Analysemethoden. Verschiedene Liganden in biotischen und abiotischen Systemen können hiermit detektiert werden. Dazu zählen folgende Organismengruppen und deren Bestandteile:
- Niedermolekulare organische und anorganische Verbindungen jedweder Art
- Umweltgifte
- Agrarchemikalien
- Hormone
- Proteine
- Antibiotika
- Neurotoxine
- Bakterien
- Viren
- Parasiten

Die Integration der Nanosensoren in ein massenfertigbares *Lab-on-Chip*-System wird durch diese Erfindung ermöglicht, welches als kompaktes, portables Analysesystem für die obengenannten Anwendungen verwendbar ist. Dadurch kann die Messung innerhalb weniger Minuten durchgeführt werden, was in ausgewählte Fällen lebensrettend sein kann. Das Detektionssystem kann als Einweg-Mikrofluidik-System verwendet werden, sodass es für jeden einzelnen Test einmalig verwendet wird. Das System kann daher in großen Stückzahlen hergestellt werden.

Die in der Beschreibung, den Ansprüchen und den Figuren offenbarten Merkmale der Erfindung können sowohl einzeln als auch in beliebiger Kombination für die Verwirklichung der Erfindung wesentlich sein.

### Bezugszeichenliste

- 50: Medium
- 110: Pore
- 112: Biorezeptoren
- 114: Biomoleküle
- 120: Membran
- 125: Detektionsbereich
- 130: Flusspfad
- 210, 220: Detektorabschnitte
- 215, 225: Kanalbereiche
- 310, 320: Stromspannungscharakteristiken
- 315: erste Elektrode
- 325: zweite Elektrode
- 500A, 500B: Detektorbereiche
- 510: Anschlusselektroden
- 520: Elektrolyt-Einlass
- 521: Kanal
- 530: Analyt-Einlässe
- 610: Substrat
- 620: Photoresistschicht
- 630: Zwischenschicht (z.B. aus Chrom)
- 640: Elektrodenschicht (z.B. aus Silber oder Gold)
- 660: Haftmittelschicht
- 670: Maskenschicht
- 680: Klebeschicht

## Patentansprüche

1. Verfahren zur Herstellung eines Detektionssystems für Biomoleküle (114) in einem Medium (50), **gekennzeichnet durch:**
Bereitstellen (S110) eines ersten Detektorabschnittes (210) mit einem ersten Kanalbereich (215) und eines zweiten Detektorabschnittes (220) mit einem zweiten Kanalbereich (225), wobei sowohl der erste Detektorabschnitt (210) als auch der zweite Detektorabschnitt (220) jeweils ein Substrat (610, 610b) umfassen und auf dem Substrat (610a) des ersten Detektorabschnitts (210) eine erste Elektrode (315) und eine zweite Elektrode (325) ausgebildet sind;
Bereitstellen (S120) einer Membran (120) mit zumindest einer Pore (110); und
Anordnen (S130) des ersten Detektorabschnittes (210) und des zweiten Detektorabschnittes (220) auf gegenüberliegenden Seiten der Membran (120), sodass zumindest ein Teil des ersten Kanalbereichs (215) und des zweiten Kanalbereichs (225) durch die Membran (120) getrennt werden und der erste Kanalbereich (215) und der zweite Kanalbereich (225) sich miteinander zu einem Kanalsystem verbinden, um einen Flusspfad (130) für das Medium (50) durch die zumindest eine Pore (110) der Membran (120) zu bilden,
wobei entlang des Flusspfades (130) durch die Membran-Biorezeptoren (112) an der Membran (120) ausgebildet sind, um eine Konzentration der Biomoleküle (114) in dem Medium (50) durch eine Messung des Flusses entlang der Flusspfades (130) zu bestimmen, wobei der Schritt des Anordnens (S130) Folgendes umfasst:
- Anordnen der Membran (120) auf dem ersten Detektorabschnitt (210) oder auf dem zweiten Detektorabschnitt (220); und danach
- Entfernen eines Teiles der Membran (120) außerhalb eines Detektionsbereiches (125).

2. Verfahren nach Anspruch 1, das weiter ein Ausbilden einer Klebeschicht (680) umfasst, die in Kontakt zu der Membran (120) steht, wobei die Klebeschicht (680) derart in Kontakt zu der Membran (120) gebracht wird, dass zumindest einige der Poren (110) durch die Klebeschicht (680) verschlossen werden, um dadurch eine Sensitivität der Membran (120) durch eine Verringerung einer Anzahl von Poren (110) für die Flussmessung des Mediums (50) zu erhöhen.

3. Verfahren nach einem der vorhergehenden Ansprüche, welches außerdem Folgendes umfasst:
Anbringen der Biorezeptoren (112) an der Membran (120) durch eine Funktionalisierung, wobei das Funktionalisieren vor oder nach dem Anordnen des ersten Detektorabschnittes (210) und des zweiten Detektorabschnittes (220) auf gegenüberliegenden Seiten der Membran (120) ausgeführt wird.

4. Verfahren nach Anspruch 3, wobei das Funktionalisieren zumindest folgende Funktionalisierungsschritte umfasst:
- Aktivieren einer Carboxy-Endgruppe, um ein Amin-reaktives Intermediat zu erhalten; und
- Amidisieren des Amin-reaktive Intermediats, um gewünschte Biorezeptoren (112) an der Membran (120) auszubilden,
wobei das Funktionalisieren in allen Bereichen der Membran (120) gleich erfolgt oder beim Funktionalisieren in verschiedenen Bereichen verschiedene Biorezeptoren (112) in den Poren (110) ausgebildet werden, sodass die Membran (120) in verschiedenen Bereichen für verschiedene Biomoleküle (114) sensitiv wird.

5. Verfahren nach einem der vorhergehenden Ansprüche, das weiter ein Laminieren der Membran (120) auf den ersten Detektorabschnitt (210) und/oder auf den zweiten Detektorabschnitt (220) umfasst.

6. Verfahren nach einem der vorhergehenden Ansprüche, wobei der erste Detektorabschnitt (210) und der zweite Detektorabschnitt (220) mit den gegenüberliegenden Seiten der Membran (120) durch eine thermische Behandlung bei einer Temperatur von zumindest 50°C oder zumindest 65°C miteinander verbunden werden.

7. Verfahren nach einem der vorhergehenden Ansprüche, wobei die Konzentration der Biomoleküle (114) in dem Medium (50) durch zumindest eine der folgenden Messungen bestimmt wird: (i) eine Flussmessung durch die zumindest eine Pore (110), (ii) eine Impedanzmessung, und (iii) eine elektrokinetische Messung, insbesondere eine Elektrophorese- oder eine Elektroosmose-Messung.

8. Verfahren nach einem der vorhergehenden Ansprüche, wobei die Biomoleküle (114) Prostata-spezifisches Antigene (PSA) sind und die Biorezeptoren (112) Aptamere umfassen, die insbesondere eine der folgenden Aptamere sind:
a) 
NH₂-C₆-CCGUCAGGUCACGGCAGCGAAGCUCUAGGCGCG
GCCAGUUGC-OH;
b) NH₂-C₆-TTTTTAATTAAAGCTCGCCATCAAATAGCTTT-OH;
c)

NH2-C6-ACGCTCGGATGCCACTACAGGTTGGGGTCGGCATGCGTC
CGGAGAAGGGCAAACGAGAGGTCACCAGCACGTCCATGAG-OH.

9. Detektionssystem für Biomoleküle (114) in einem Medium (50), wobei das Detektionssystem Folgendes umfasst:
einen ersten Detektorabschnitt (210) mit einem ersten Kanalbereich (215) und einen zweiten Detektorabschnittes (220) mit einem zweiten Kanalbereich (225), wobei sowohl der erste Detektorabschnitt (210) als auch der zweite Detektorabschnitt (220) jeweils ein Substrat (610, 610b) umfassen und auf dem Substrat (610a) des ersten Detektorabschnitts (210) eine erste Elektrode (315) und eine zweite Elektrode (325) ausgebildet sind und in den ersten Kanalbereich (215) und den zweiten Kanalbereich (225) das Medium (50) einbringbar ist;
eine erste Haftmittelschicht (660a) auf dem Substrat (610a) des ersten Detektorabschnittes (210) und eine zweite Haftmittelschicht (660b) auf dem Substrat (610b) des zweiten Detektorabschnittes (220);
eine erste Maskenschicht (670a) auf der ersten Haftmittelschicht (660a) und eine zweite Maskenschicht (670b) auf der zweiten Haftmittelschicht (660b);
eine erste Klebeschicht (680a) auf der ersten Maskenschicht (670a) und eine zweite Klebeschicht (680b) auf der zweiten Maskenschicht (670b);
eine Membran (120), die zumindest eine Pore (110) aufweist und den ersten Kanalbereich (215) von dem zweiten Kanalbereich (225) trennt und fluid zwischen der ersten Elektrode (315) und der zweiten Elektrode (325) angeordnet ist, wobei die Membran (120) zwischen der ersten Klebeschicht (680a) und der zweiten Klebeschicht (680b) ausgebildet ist und die erste und zweite Klebeschichten (680a, 680b) auch außerhalb des Detektionsbereiches (125) trennt,
wobei an oder in der Pore (110) Biorezeptoren (112) ausgebildet sind, die unter anderem eines der folgenden Aptamere umfassen:
(i) 
NH₂-C₆-CCGUCAGGUCACGGCAGCGAAGCUCUAGGCGCG
GCCAGUUGC-OH;
(ii) NH₂-C₆-TTTTTAATTAAAGCTCGCCATCAAATAGCTTT-OH;
(iii) 
NH₂-C₆-ACGCTCGGATGCCACTACAGGTTGGGGTCGG
GCATGCGTCCGGAGAAGGGCAAACGAGAGGTCAC
CAGCACGTCCATGAG-OH,
 sodass eine PSA-Konzentration im Medium (50) über eine Widerstandsmessung entlang des Flusspfades (130) für das Medium (50) zwischen der ersten Elektrode (315) und der zweiten Elektrode (325) messbar ist.

10. Detektionssystem nach Anspruch 9, wobei die zumindest eine Pore (110) in der Membran (120) ein konisch zulaufendes oder zylindrisches Profil entlang des Flusspfades (130) aufweist.

11. Detektionssystem nach Anspruch 9 oder Anspruch 10, wobei die Membran in verschiedenen Bereichen verschiedene Rezeptoren (112) oder Aptamere umfasst, um eine simultane Detektion verschiedener Biomoleküle (114) zu ermöglichen.

12. Detektionssystem nach einem der Ansprüche 9 bis 11, wobei der erste Kanalbereich (215) und/oder der zweite Kanalbereich (225) senkrecht zum Flusspfad (130) eine maximale Kanalbreite von bis zu 1 mm oder bis zu 50 µm oder höchstens 10 µm aufweisen.

13. Detektionssystem nach einem der Ansprüche 9 bis 12, das weiter einen Elektrolyt-Einlass (520) bei der zweiten Elektrode (325) und einen Analyt-Einlass (530) bei der ersten Elektrode (315) umfasst, um das Medium (50) in dem Analyt-Einlass (530) und einen Elektrolyten in den Elektrolyt-Einlass (520) einbringen zu können, um so eine für die Detektion erforderliche Menge des Mediums (50) zu verringern.

14. Verwendung eines Detektionssystems nach einem der Ansprüche 9 bis 13 außerhalb des menschlichen oder tierischen Körpers zur Detektion von Biomolekülen (114) in einem Medium (50) durch ein Messen einer elektrischen Größe, die von einem elektrischen Widerstand zwischen der Elektrode (315) und der zweiten Elektrode (325) abhängt.

## Claims

1. Method for producing a detection system for biomolecules (114) in a medium (50), **characterized by**:
providing (S110) a first detector portion (210) having a first channel region (215) and a second detector portion (220) having a second channel region (225), both the first detector portion (210) and the second detector portion (220) having a particular substrate (610, 610b), and a first electrode (315) and a second electrode (325) being formed on the substrate (610a) of the first detector portion (210);
providing (S120) a membrane (120) having at least one pore (110); and
arranging (S130) the first detector portion (210) and the second detector portion (220) on opposite sides of the membrane (120) such that at least part of the first channel region (215) and of the second channel region (225) are separated by the membrane (120) and the first channel region (215) and the second channel region (225) are interconnected to form a channel system in order to form a flow path (130) for the medium (50) through the at least one pore (110) of the membrane (120),
bioreceptors (112) being formed on the membrane (120) along the flow path (130) through the membrane in order to determine a concentration of the biomolecules (114) in the medium (50) by measuring the flow along the flow path (130), the step of arranging (S130) comprising the following:
- arranging the membrane (120) on the first detector portion (210) or on the second detector portion (220); and then
- removing part of the membrane (120) outside a detection region (125).

2. Method according to claim 1, further comprising forming an adhesive layer (680) in contact with the membrane (120), the adhesive layer (680) being brought into contact with the membrane (120) in such a way that at least some of the pores (110) are closed by the adhesive layer (680), thereby increasing the sensitivity of the membrane (120) by reducing the number of pores (110) for the flow measurement of the medium (50).

3. Method according to either of the preceding claims, which method further comprises the following:
attaching the bioreceptors (112) to the membrane (120) by means of a functionalization, the functionalization being carried out before or after the arrangement of the first detector portion (210) and the second detector portion (220) on opposite sides of the membrane (120).

4. Method according to claim 3, wherein the functionalization comprises at least the following functionalization steps:
- activating a carboxyl end group to obtain an amine-reactive intermediate; and
- amidizing the amine-reactive intermediate to form desired bioreceptors (112) on the membrane (120),
wherein the functionalization takes place in the same way in all regions of the membrane (120), or different bioreceptors (112) are formed in the pores (110) in different regions during the functionalization such that the membrane (120) becomes sensitive to different biomolecules (114) in different regions.

5. Method according to any of the preceding claims, further comprising laminating the membrane (120) on the first detector portion (210) and/or on the second detector portion (220).

6. Method according to any of the preceding claims, wherein the first detector portion (210) and the second detector portion (220) are interconnected by means of the opposite sides of the membrane (120) by a thermal treatment at a temperature of at least 50°C or at least 65°C.

7. Method according to any of the preceding claims, wherein the concentration of the biomolecules (114) in the medium (50) is determined by at least one of the following measurements: (i) a flow measurement through the at least one pore (110), (ii) an impedance measurement, and (iii) an electrokinetic measurement, in particular an electrophoresis or an electroosmosis measurement.

8. Method according to any of the preceding claims, wherein the biomolecules (114) comprise prostate-specific antigens (PSA) and the bioreceptors (112) comprise aptamers, which are in particular one of the following aptamers:
a) NH₂-C₆-CCGUCAGGUCACGGCAGCGAAGCUCUAGGCGCGGCCAGUUGC-OH;
b) NH₂-C₆-TTTTTAATTAAAGCTCGCCATCAAATAGCTTT-OH; and
c)

NH2-C6-
ACGCTCGGATGCCACTACAGGTTGGGGTCGGGCATGCGTCCGGAGAAGGGCA
AACGAGAGGTCACCAGCACGTCCATGAG-OH.

9. Detection system for biomolecules (114) in a medium (50), wherein the detection system comprises the following:
a first detector portion (210) having a first channel region (215) and a second detector portion (220) having a second channel region (225), wherein both the first detector portion (210) and the second detector portion (220) have a particular substrate (610, 610b), and a first electrode (315) and a second electrode (325) are formed on the substrate (610a) of the first detector portion (210), and the medium (50) can be introduced into the first channel region (215) and the second channel region (225);
a first adhesive layer (660a) on the substrate (610a) of the first detector portion (210) and a second adhesive layer (660b) on the substrate (610b) of the second detector portion (220);
a first mask layer (670a) on the first adhesive layer (660a) and a second mask layer (670b) on the second adhesive layer (660b);
a first adhesive layer (680a) on the first mask layer (670a) and a second adhesive layer (680b) on the second mask layer (670b);
a membrane (120) which has at least one pore (110) and separates the first channel region (215) from the second channel region (225) and is fluidically arranged between the first electrode (315) and the second electrode (325), wherein the membrane (120) is formed between the first adhesive layer (680a) and the second adhesive layer (680b) and also separates the first and second adhesive layers (680a, 680b) outside the detection region (125),
wherein bioreceptors (112) are formed on or in the pore (110) and include, among other things, one of the following aptamers:
(i) NH₂-C₆-CCGUCAGGUCACGGCAGCGAAGCUCUAGGCGCG GCCAGUUGC-OH;
(ii) NH₂-C₆-TTTTTAATTAAAGCTCGCCATCAAATAGCTTT-OH; and
(iii) 
NH2-C6-
ACGCTCGGATGCCACTACAGGTTGGGGTCGGGCATGCGTCCGGAGAAGGGCA
AACGAGAGGTCAC CAGCACGTCCATGAG-OH,
such that a PSA concentration in the medium (50) can be measured via a resistance measurement along the flow path (130) for the medium (50) between the first electrode (315) and the second electrode (325).

10. Detection system according to claim 9, wherein the at least one pore (110) in the membrane (120) has a tapered or cylindrical profile along the flow path (130).

11. Detection system according to either claim 9 or claim 10, wherein the membrane comprises different receptors (112) or aptamers in different regions in order to enable simultaneous detection of different biomolecules (114).

12. Detection system according to any of claims 9 to 11, wherein the first channel region (215) and/or the second channel region (225) has a maximum channel width of up to 1 mm or up to 50 µm or at most 10 µm perpendicular to the flow path (130).

13. Detection system according to any of claims 9 to 12, further comprising an electrolyte inlet (520) at the second electrode (325) and an analyte inlet (530) at the first electrode (315) in order to be able to introduce the medium (50) in the analyte inlet (530) and an electrolyte into the electrolyte inlet (520), in order to reduce the amount of medium (50) required for the detection.

14. Use of a detection system according to any of claims 9 to 13 outside of the human or animal body for the detection of biomolecules (114) in a medium (50) by measuring an electrical variable which is a function of an electrical resistance between the electrode (315) and the second electrode (325).

## Revendications

1. Procédé de fabrication d'un système de détection de biomolécules (114) dans un milieu (50), **caractérisé par** :
la fourniture (S110) d'une première section de détecteur (210) comportant une première région de canal (215) et d'une seconde section de détecteur (220) comportant une seconde région de canal (225), à la fois la première section de détecteur (210) et la seconde section de détecteur (220) comprenant respectivement un substrat (610, 610b) et une première électrode (315) et une seconde électrode (325) étant formées sur le substrat (610a) de la première section de détecteur (210) ;
la fourniture (S120) d'une membrane (120) comportant au moins un pore (110) ; et
l'agencement (S130) de la première section de détecteur (210) et de la seconde section de détecteur (220) sur des côtés opposés de la membrane (120), de sorte qu'au moins une partie de la première région de canal (215) et de la seconde région de canal (225) sont séparées par la membrane (120) et la première région de canal (215) et la seconde région de canal (225) sont reliées l'une à l'autre pour former un système de canaux afin de former un trajet d'écoulement (130) pour le milieu (50) à travers l'au moins un pore (110) de la membrane (120),
des biorécepteurs (112) étant formés sur la membrane (120) le long du trajet d'écoulement (130) à travers la membrane afin de déterminer une concentration des biomolécules (114) dans le milieu (50) en mesurant l'écoulement le long du trajet d'écoulement (130), l'étape d'agencement (S130) comprenant :
- l'agencement de la membrane (120) sur la première section de détecteur (210) ou sur la seconde section de détecteur (220) ; et ensuite
- l'élimination d'une partie de la membrane (120) à l'extérieur d'une région de détection (125).

2. Procédé selon la revendication 1, comprenant en outre la formation d'une couche adhésive (680) en contact avec la membrane (120), la couche adhésive (680) étant amenée en contact avec la membrane (120) de sorte qu'au moins certains des pores (110) sont fermés par la couche adhésive (680) afin d'augmenter ainsi une sensibilité de la membrane (120) en réduisant le nombre de pores (110) pour la mesure d'écoulement du milieu (50).

3. Procédé selon l'une des revendications précédentes, comprenant en outre :
la fixation des biorécepteurs (112) à la membrane (120) par fonctionnalisation, la fonctionnalisation étant effectuée avant ou après l'agencement de la première section de détecteur (210) et de la seconde section de détecteur (220) sur des côtés opposés de la membrane (120).

4. Procédé selon la revendication 3, la fonctionnalisation comprenant au moins les étapes de fonctionnalisation suivantes :
- activation d'un groupe terminal carboxy afin d'obtenir un intermédiaire réactif avec les amines ; et
- amidation de l'intermédiaire réactif aux amines afin de former les biorécepteurs (112) souhaités sur la membrane (120),
la fonctionnalisation étant effectuée de la même manière dans toutes les régions de la membrane (120) ou différents biorécepteurs (112) étant formés dans les pores (110) lors de la fonctionnalisation dans différentes régions, de sorte que la membrane (120) est sensible à différentes biomolécules (114) dans différentes régions.

5. Procédé selon l'une des revendications précédentes, comprenant en outre une stratification de la membrane (120) sur la première section de détecteur (210) et/ou sur la seconde section de détecteur (220).

6. Procédé selon l'une des revendications précédentes, la première section de détecteur (210) et la seconde section de détecteur (220) étant reliées l'une à l'autre avec les côtés opposés de la membrane (120) par un traitement thermique à une température d'au moins 50 °C ou d'au moins 65 °C.

7. Procédé selon l'une des revendications précédentes, la concentration des biomolécules (114) dans le milieu (50) étant déterminée par au moins une des mesures suivantes : (i) une mesure d'écoulement à travers l'au moins un pore (110), (ii) une mesure d'impédance, et (iii) une mesure électrocinétique, en particulier une mesure par électrophorèse ou une mesure par électro-osmose.

8. Procédé selon l'une des revendications précédentes, les biomolécules (114) étant des antigènes prostatiques spécifiques (PSA) et les biorécepteurs (112) comprenant des aptamères, lesquels sont en particulier l'un des aptamères suivants :
a) NH₂-C₆-CCGUCAGGUCACGGCAGCGAAGCUCUAGGCGCGGCCAGUUGC-OH ;
b) NH₂-C₆-TTTTTAATTAAAGCTCGCCATCAAATAGCTTT-OH ;
c)

NH2-C6-
ACGCTCGGATGCCACTACAGGTTGGGGTCGGGCATGCGTCCGGAGAAGGGCAAAC
GAGAGGTCACCAGCACGTCCATGAG-OH.

9. Système de détection de biomolécules (114) dans un milieu (50), le système de détection comprenant:
une première section de détecteur (210) comportant une première région de canal (215) et une seconde section de détecteur (220) comportant une seconde région de canal (225), à la fois la première section de détecteur (210) et la seconde section de détecteur (220) comprenant respectivement un substrat (610, 610b) et une première électrode (315) et une seconde électrode (325) étant formées sur le substrat (610a) de la première section de détecteur (210) et le milieu (50) pouvant être amené dans la première région de canal (215) et la seconde région de canal (225) ;
une première couche de collage (660a) sur le substrat (610a) de la première section de détecteur (210) et une seconde couche de collage (660b) sur le substrat (610b) de la seconde section de détecteur (220) ;
une première couche de masquage (670a) sur la première couche de collage (660a) et une seconde couche de masquage (670b) sur la seconde couche de collage (660b) ;
une première couche adhésive (680a) sur la première couche de masquage (670a) et une seconde couche adhésive (680b) sur la seconde couche de masquage (670b) ;
une membrane (120) présentant au moins un pore (110) et séparant la première région de canal (215) de la seconde région de canal (225) et étant agencée fluidiquement entre la première électrode (315) et la seconde électrode (325), la membrane (120) étant formée entre la première couche adhésive (680a) et la seconde couche adhésive (680b) et séparant en outre les première et seconde couches adhésives (680a, 680b) à l'extérieur de la région de détection (125),
des biorécepteurs (112) étant formés sur ou dans le pore (110) et comprenant entre autres l'un des aptamères suivants :
(i) 
NH2-C6-CCGUCAGGUCACGGCAGCGAAGCUCUAGGCGCGGCCAGUUGC-
OH ;
(ii) NH₂-C₆-TTTTTAATTAAAGCTCGCCATCAAATAGCTTT-OH ;
(iii) 
NH2-C6-
ACGCTCGGATGCCACTACAGGTTGGGGTCGGGCATGCGTCCGGAGAAGGG
CAAACGAGAGGTCACCAGCACGTCCATGAG-OH,
 de sorte qu'une concentration de PSA dans le milieu (50) peut être mesurée par l'intermédiaire d'une mesure de résistance le long du trajet d'écoulement (130) pour le milieu (50) entre la première électrode (315) et la seconde électrode (325).

10. Système de détection selon la revendication 9, l'au moins un pore (110) dans la membrane (120) présentant un profil conique ou cylindrique le long du trajet d'écoulement (130).

11. Système de détection selon la revendication 9 ou la revendication 10, la membrane comprenant différents récepteurs (112) ou aptamères dans différentes régions afin de permettre une détection simultanée de différentes biomolécules (114).

12. Système de détection selon l'une des revendications 9 à 11, la première région de canal (215) et/ou la seconde région de canal (225) présentant une largeur de canal maximale perpendiculaire au trajet d'écoulement (130) et allant jusqu'à 1 mm ou jusqu'à 50 µm ou d'au plus 10 µm.

13. Système de détection selon l'une des revendications 9 à 12, comprenant en outre une entrée d'électrolyte (520) au niveau de la seconde électrode (325) et une entrée d'analyte (530) au niveau de la première électrode (315) afin de pouvoir amener le milieu (50) dans l'entrée d'analyte (530) et un électrolyte dans l'entrée d'électrolyte (520) de manière à réduire une quantité du milieu (50) nécessaire à la détection.

14. Utilisation d'un système de détection selon l'une des revendications 9 à 13 à l'extérieur du corps humain ou animal permettant la détection de biomolécules (114) dans un milieu (50) en mesurant une grandeur électrique qui dépend d'une résistance électrique entre l'électrode (315) et la seconde électrode (325).
